# EUROPÄISCHE PATENTANMELDUNG

(11) **EP 4 763 165 A1**
(43) Veröffentlichungstag der Anmeldung: **24.06.2026**
(21) Anmeldenummer: 24020351.3
(22) Anmeldetag: 20.12.2024
(51) Int. Cl.: A61F 13/08, D04B 1/26

(54) **BEINBEKLEIDUNGSSTÜCK**

(71) Anmelder: medi GmbH & Co. KG, 95448 Bayreuth (DE)
(72) Erfinder: Huger, Christa, 95326 Kulmbach (DE)

(57) **Zusammenfassung**

Beinbekleidungsstück (1, 1', 1") aufweisend ein zumindest ein Fußteil ausbildendes Grundgestrickteil (2, 2', 2", 2‴, 2"") mit zumindest einer ersten in das Grundgestrickteil (2, 2', 2", 2‴, 2"") eingestrickten Funktionszone (3, 3', 3", 3‴, 3ʺʺ), welche zur Aufnahme einer Ferse eines Fußes ausgebildet ist, wobei in die erste Funktionszone (3, 3', 3", 3‴, 3"") zumindest abschnittsweise zumindest ein erster Haftfaden (4) eingestrickt ist, um dem Beinbekleidungsstück (1, 1', 1") in einem Fersenbereich eine rutschhemmende Wirkung zu verleihen.

## Beschreibung

Die vorliegende Erfindung bezieht sich auf ein Beinbekleidungsstück, insbesondere mit Haftwirkung, nach dem Oberbegriff des Anspruchs 1.

Derartige Beinbekleidungsstücke werden insbesondere bei sportlichen Aktivitäten eingesetzt. Sie finden aber auch Anwendung in der Medizin. Bei Ausbildung des Beinbekleidungsstücks als kompressives Kleidungsstück dient dieses dazu gezielt Druck auf den Körper eines Patienten auszuüben. Der auf den Körper eines Patienten ausgeübte Druck wird als Kompression bezeichnet. Ziel bei Kompressionsstrümpfen beispielsweise, insbesondere in Form von Wadenstrümpfen, ist es unter anderem ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von kompressiven Beinbekleidungsstücken in dem Sportbereich bewirken diese eine Leistungssteigerung bzw. eine verbesserte Regeneration.

Zur Ausbildung dieser Beinbekleidungsstücke werden diese vorzugsweise mittels einer Rundstrickmaschine oder einer Flachstrickmaschine flach- oder rundgestrickt. Hierzu werden die Beinbekleidungsstücke, insbesondere deren Grundgestrickteile, üblicherweise aus wenigstens einem maschenbildenden Strickfaden und einem eingelegten elastischen Schussfaden gestrickt. Zur Herstellung, insbesondere zur Messung und Gütesicherung, von kompressiven Arm- oder Beinstrümpfen für die medizinische Anwendung, existiert die Norm RAL-GZ 387 der Gütezeichengemeinschaft. Aus den Prüfbestimmungen der RAL kann entnommen werden, wie der Druck eines Kompressionsstrumpfes auf ein Bein zu bestimmen ist. Als Messmittel, insbesondere Kompressionsprüfgerät, wird die Prüfung am HOSY Messgerät (Institut Hohenstein) vorgeschlagen. Die Prüfung erfolgt anhand der Messung der Spannkraft in den mehreren Messpunkten, welche abhängig von der jeweiligen Dehnbarkeit des Gestricks, also abhängig von der Elastizität des Gestricks, variiert. Aus der Spannkraft wird die Kompression errechnet. D.h. je mehr Elastizität ein Gestrick in Gestrickquerrichtung aufweist, desto geringer ist die durch das Gestrick am Patienten ausgeübte Kompression. Andererseits je geringer die Elastizität bei einer definierten Zugkraft des Gestrickteils ist, desto höher ist die am Patienten ausgeübte Kompression.

Aus dem Stand der Technik sind eine Vielzahl von Beinbekleidungsstücken, insbesondere kompressive Kleidungsstücke für Beine, bekannt. Ein Beinbekleidungsstück, insbesondere ausgebildet als Kompressionsstrumpf, mit rutschhemmender Wirkung ist beispielsweise aus der US 4,149,274 bekannt.

Dieser bekannte Kompressionsstrumpf, welcher insbesondere für bettlägerige Patienten entwickelt wurde, umfasst insbesondere einen rutschfesten unteren Sohlenabschnitt, welcher sich zwischen einer Fersentasche und einem Zehenabschnitt erstreckt. Die rutschhemmende Wirkung wird dem Kompressionsstrumpf dabei durch das Einstricken eines Garns mit hohem Reibungskoeffizienten, wie z. B. blankem Elasthan, verliehen. Das Garn wird während des Strickprozesses mit dem Grundgarn des Kompressionsstrumpfes verstrickt und dabei bevorzugt mittels der Strickbindung Fang und Flottung, insbesondere über Fangmaschen, in das Grundgestrick eingearbeitet. Um die Fangmaschen und Flottungen des Reibungsgarns auf der Außenseite der Sohle zu positionieren wird der Kompressionsstrumpf nach dem Strickvorgang umgestülpt. Teile der Fangmaschen des Reibungsgarns ragen dadurch an der Außenseite des Sohlenabschnitts heraus und kommen in einem Tragezustand mit einem Fußboden in Kontakt und verringern dadurch das Ausrutschen des Fußes des Trägers. Im Vergleich zu separat aufgebrachten Antirutschelementen bringt das Einstricken eines Reibungsgarns den Vorteil mit sich, dass die Dehnbarkeit des Kompressionsstrumpfes nicht übermäßig eingeschränkt wird.

Als nachteilig der aus diesem Stand der Technik bekannten Ausgestaltung des Beinbekleidungsstücks, insbesondere Kompressionsstrumpfes, erweist sich die Anordnung bzw. das Einstricken des Reibungsgarns in dem Mittelfußbereich an der Fußsohle des Beinbekleidungsstücks. Beim Gehen findet stets ein Abrollen des Fußes statt. Das natürliche Abrollverhalten weist folgende Schritte auf: Aufsetzen der Ferse, vollständiger Bodenkontakt, Abdrücken, Bewegung nach vorne, erneutes Aufsetzen der Ferse. Durch das Einstricken des Reibungsgarns zwischen der Fersentasche und dem Zehenabschnitt wird zwar eine rutschfester Sohlenabschnitt zur Verfügung gestellt. Da dieser aber nur in einem Mittelfußbereich ausgebildet wird, hilft dieser unter Berücksichtigung der natürlichen Abrollbewegung nur begrenzt, um ein Ausrutschen zu vermeiden. Insbesondere für einen ersten Kontakt mit einem Boden, also bei dem Aufsetzen der Ferse auf den Boden, wird bei diesem aus dem Stand der Technik bekannten Kompressionsstrumpf kein rutschfester Sohlenabschnitt zur Verfügung gestellt.

Einen weiteren Nachteil des bekannten Stands der Technik stellt die Ausbildung der rutschfesten Oberfläche nur an einer Seite des Gestricks, insbesondere an der Außenseite dar. Die vorgeschlagene Einbindung des Reibungsgarns, also das Einstricken des Garns durch Fang und Flottung, insbesondere über Fangmaschen, in das Grundgestrick ermöglicht nämlich nur diese einseitige Ausbildung der rutschfesten Oberfläche an dem Gestrick. Beispielsweise wird bei der Ausbildung von Flottungen, wie im Stand der Technik vorgeschlagen, das Reibungsgarn über eine oder mehrere Grundstrickmaschen geflottet und kommt somit oberhalb dieser Maschen zum Liegen.

Dadurch tritt das Reibungsgarn an der Oberfläche hervor. Innerhalb des Herstellungsprozesses des Kompressionsstrumpfes muss somit entschieden werden, ob diese rutschfeste Oberfläche an der Innenseite oder Außenseite des Kompressionsstrumpfes zum Liegen kommen soll. Ist es gewünscht, dass diese die Außenseite bildet, dann muss der Kompressionsstrumpf in einem zusätzlichen Fertigungsschritt noch umgestülpt werden, da, wie aus dem Stand der Technik bekannt, diese bei einem Strickprozess an der Innenseite des Gestricks gebildet wird. Nachteilig dabei ist, dass dieser meist manuelle Schritt zusätzlichen Fertigungsaufwand und somit zusätzliche Fertigungskosten mit sich bringt.

Zudem erweist sich dabei als nachteilig, dass die eigentliche Innenseite des Kompressionsstrumpfes die Außenseite bildet. Bei kompressiven Beinbekleidungsstücken ist es üblich, dass einer oder mehrere kompressive Fäden, insbesondere Schussfäden, in das Gestrick, insbesondere in einen Fessel- und Wadenbereich, eingearbeitet werden, dies üblicherweise ebenfalls über Fang und Flottung. Diese Fäden treten somit nach dem Umstülpen des Strumpfes ebenfalls an der Gestrickaußenseite hervor. Da diese kompressiven Fäden üblicherweise ebenfalls aus Elasthan bestehen, wie das zuvor beschriebene Reibungsgarn, weist das kompressive Gestrick, insbesondere Beinbekleidungsstück, zwangsläufig auch in den kompressiven Abschnitten, insbesondere in dem Fessel- und Wadenabschnitt an der Außenseite eine rutschfeste Oberfläche auf. Dies bringt den Nachteil mit sich, dass auch in gegebenenfalls unerwünschten Gestrickabschnitten an der Außenseite eine rutschfeste Oberfläche gebildet wird. Insbesondere im Bereich der Wade würde eine derartige Ausbildung dazu führen, dass die Anziehbarkeit eines weiteren Kleidungsstücks oberhalb des Kompressionsstrumpfes deutlich erschwert wird.

Schließlich ist auch ein Nachteil des aus diesem Stand der Technik bekannten Beinbekleidungsstücks, dass der Kompressionsstrumpf nur für bettlägerige Patienten ausgebildet ist. Der Kompressionsstrumpf weist daher nur an der Fußsohle, insbesondere in einem Mittelfußbereich, eine rutschfeste Oberfläche auf.

Der vorliegenden Erfindung liegt daher die Aufgabe zugrunde ein Beinbekleidungsstück zu schaffen, welches die Nachteile aus dem Stand der Technik vermeidet, insbesondere die Haftwirkung des Beinbekleidungsstücks gegenüber mehreren externen Oberflächen deutlich verbessert.

Gemäß einem Ausführungsbeispiel des Beinbekleidungsstücks weist dieses zumindest ein Fußteil ausbildendes Grundgestrickteil mit zumindest einer ersten in das Grundgestrickteil eingestrickten Funktionszone auf, welche zur Aufnahme einer Ferse eines Fußes ausgebildet ist, wobei in die erste Funktionszone zumindest abschnittsweise zumindest ein erster Haftfaden eingestrickt ist, um dem Beinbekleidungsstück in einem Fersenbereich eine rutschhemmende Wirkung zu verleihen.

Bei dem Haftfaden handelt es sich bevorzugt um einen Silikon-, Polyurethan-, Polyester-, Elastan- oder Kautschukfaden. Wird ein Polyurethanfaden verwendet, dann besteht dieser bevorzugt aus einem thermoplastischen Polyurethan. Der Kautschukfaden besteht wiederum bevorzugt aus Gummi oder Neopren. Der Haftfaden kann dabei als Singlefaden oder Filamentgarn ausgebildet sein. Ist der Faden als Singlefaden ausgebildet, dann weist dieser bevorzugt einen Fadenkern, bevorzugt aus Elastan oder Polyamid, auf, wobei der Fadenkern mit den zuvor genannten Materialien beschichtet oder umwunden ist.

Zur besseren Verstrickbarkeit ist der Faden ferner bevorzugt mit einer fluidlöslichen, insbesondere wasserlöslichen, Beschichtung versehen oder mit einem fluidlöslichen, insbesondere wasserlöslichen, Umwindefaden umwunden. Ein solcher Umwindefaden oder Beschichtung reduziert die Reibung des ansonsten haftenden Silikonmaterials während des Strickvorgangs, insbesondere an den maschenbildenden Elementen (Nadel, Fadenführer etc.) und der Fadenzuführung. Nach dem Stricken wird das Gestrickteil bevorzugt gewaschen, so dass sich der wasserlösliche Umwindefaden oder die Beschichtung auflöst und die haftenden Eigenschaften des Haftfadens voll zur Wirkung kommt.

Gemäß einem zweiten Ausführungsbeispiel tritt der zumindest eine eingestrickte erste Haftfaden an der Gestrickinnen- und/ oder Außenseite der ersten Funktionszone hervor. D.h. er ragt bevorzugt aus dem Gestrick bzw. der ersten Funktionszone heraus. Dazu ist der Haftfaden bevorzugt auf einen Grundstrickfaden an der Innen- und/ oder Außenseite plattiert. Alternativ kann der Haftfaden auch ohne den Grundstrickfaden über mehrere Maschenstäbchen oder Maschenreihen hinweg in die erste Funktionszone eingestrickt sein. Der Haftfaden tritt besonders bevorzugt an beiden Seiten, also der Innen- und Außenseite des Beinbekleidungsstücks, hervor. Das Beinbekleidungsstück gewährleistet dadurch einen sicheren Halt gegenüber dem Träger als auch einer externen Oberfläche, wie zum Beispiel einem Boden oder einem Schuh.

Gemäß einem dritten Ausführungsbeispiel ist der zumindest eine erster Haftfaden zumindest abschnittsweise im Bereich der Fersenunterseite und/ oder der Fersenrückseite in die erste Funktionszone eingestrickt. Dabei dient der Haftfaden an der Fersenunterseite dazu einen besseren Halt gegenüber einem Boden oder einer Sohle in einem Schuh zu gewährleisten. Der Haftfaden an der Fersenrückseite dient dazu das Herausschlüpfen aus einem Schuh zur vermeiden, zumindest den Halt in einem Schuh zu verbessern. Somit ist besonders bevorzugt der Haftfaden in beiden Bereichen, also der Fersenunterseite, als auch der Fersenrückseite, in die erste Funktionszone eingestrickt.

Gemäß einem vierten Ausführungsbeispiel ist der zumindest eine erster Haftfaden im Bereich der Ferseninnenseite und/ oder der Fersenaußenseite in die erste Funktionszone eingestrickt. Dadurch und insbesondere in Kombination mit der Anordnung des Haftfadens im Bereich der Fersenrückseite, wie zuvor beschrieben, wird der Halt in einem Schuh nochmals verbessert und die Gefahr des Herausschlüpfens aus dem Schuh weiter minimiert. Auf Grund dessen ist der Haftfaden besonders bevorzugt an der Fersenrückseite sowie an der Ferseninnen- und der Fersenaußenseite eingestrickt.

Gemäß einem weiteren Ausführungsbeispiel variiert die Menge des zumindest einen ersten Haftfaden in der ersten Funktionszone in Gestricklängs- und/ oder Umfangsrichtung. Dadurch ist es möglich die Haftwirkung schritt- bzw. stufenweise zu erhöhen oder zu reduzieren. Ein abrupter Übergang zwischen Abschnitten mit und ohne Haftwirkung wird dadurch vermieden.

Gemäß einem weiteren Ausführungsbeispiel tritt der Haftfaden zur Bildung eines oder mehrerer Antirutschelemente lokal begrenzt an der Gestrickinnen- und/ oder Außenseite der ersten Funktionszone hervor. Bevorzugt weist das eine oder die mehreren Antirutschelemente jeweils eine Ausdehnung in Gestricklängs- und/ oder Umfangsrichtung zwischen 1 und 2 cm, maximal jedoch 3 cm, auf. Hierbei ist der Haftfaden in Umfangsrichtung bevorzugt nur partiell eingestrickt. Alternativ kann der Haftfaden abschnittsweise in die erste Funktionszone eingestrickt sein. Dann ist der Haftfaden in der ersten Funktionszone bevorzugt abwechselnd im Grundgestrick auf einen Grundstrickfaden plattiert und hinterlegt, um mehrere nebeneinander angeordnete Antirutschelemente zu bilden.

Gemäß einem weiteren Ausführungsbeispiel ist in das Grundgestrickteil außerhalb der ersten Funktionszone zumindest ein zweiter Haftfaden eingestrickt, um dem Beinbekleidungsstück, bevorzugt in einem Bundabschnitt, ebenfalls eine rutschhemmende Wirkung zu verleihen. Der zweite Haftfaden ist dabei bevorzugt auch ein Silikon-, Polyurethan-, Polyester-, Elastan- oder Kautschukfaden. Somit können der erste und zweite Haftfaden aus identischen Materialen gebildet sein. Alternativ unterscheiden sie sich entsprechend der vorgeschlagenen Materialien.

Neben der Einarbeitung des zweiten Haftfadens in einem Bundabschnitt, insbesondere an einer Gestrickinnenseite, um einen besseren Halt des Beinbekleidungsstücks am Fuß oder Bein eines Trägers zu gewährleisten, kann der zweite Haftfaden auch an der Außen- oder Innenseite des Beinbekleidungsstücks in einem Mittelfuß und/ oder Vorfußbereich in das Grundgestrickteil eingestrickt sein. Dadurch wird die rutschhemmende Wirkung im Sohlenbereich des Beinbekleidungsstücks weiter verbessert. Somit treten der erste Haftfaden und der zumindest eine zweite Haftfaden bevorzugt an den gegenüberliegenden Seiten des Grundgestrickteils hervor. Besonders bevorzugt tritt der erste Haftfaden in der ersten Funktionszone im Bereich der Ferse an der Gestrickaußenseite hervor und der zweite Haftfaden im Bundabschnitt oder dem Mittelfuß und/ oder Vorfußbereich an der Gestrickinnenseite.

Gemäß einem weiteren Ausführungsbeispiel umfasst das Grundgestrickteil zumindest eine zweite in das Grundgestrickteil eingestrickte Funktionszone, welche bevorzugt eine höhere Elastizität in Gestricklängs- und/ oder Umfangsrichtung, als das Grundgestrickteil aufweist. Die zweite Funktionszone dient dabei bevorzugt als Entlastungszone. Alternativ kann die zweite Funktionszone auch polsternde Eigenschaften aufweisen. Hierzu ist bevorzugt ein Plüschfaden eingestrickt. Zudem kann die zweite Funktionszone auch durch eingestrickte Zusatzmaschen gebildet sein. Dadurch kann die Passform des Beinbekleidungsstücks weiter optimiert werden.

Das Grundgestrickteil nach den vorherigen Ausführungsbeispielen ist bevorzugt durch zumindest einen maschenbildenden Grundstrickfaden sowie zumindest einen eingelegten und/ oder verstrickten elastischen Kompressionsfaden gebildet. Bei dem Kompressionsfaden handelt es sich bevorzugt um einen in das Grundgestrickteil über Fang und Flottung eingelegten Schussfaden. Das Beinbekleidungsstück wird dabei entweder auf einer Rundstrickmaschine rundgestrickt oder auf einer Flachstrickmaschine rund- oder flachgestrickt. Es werden dadurch Rechts-Links, Rechts-Rechts, Links-Links oder Halbschlauchgestricke gefertigt.

Die erste Funktionszone nach den vorherigen Ausführungsbeispielen ist bevorzugt durch den zumindest einen maschenbildenden Grundstrickfaden und den zumindest einen ersten Haftfaden oder durch den zumindest einen ersten Haftfaden alleine gebildet. Hierbei ist die erste Funktionszone bevorzugt als Pendelferse, Schlauchferse oder durch einen oder mehrere Spickel ausgebildet, wobei der zumindest eine erste Haftfaden auf den zumindest einen Grundstrickfaden zumindest abschnittsweise plattiert ist. Bei der Pendelferse wird die Fersenform durch Zu- und Abnehmen der Maschen erzeugt. Bei einer Schlauch- bzw. Beulenferse hingegen wird die Fersenform durch die Vergrößerung der Grundstrickmaschen und dem kompressionsgebenden Faden, insbesondere einem Schussfaden, der mit weniger Vorspannung in das Grundgestrickteil eingearbeitet ist, gebildet. Alternativ sind in die durch den zumindest einen Grundstrickfaden erzeugte erste Funktionszone mehrere durch den zumindest einen Haftfaden gebildete Teilmaschenreihen eingestrickt. Somit kann die erste Funktionszone entweder vollständig umlaufend oder nur bevorzugt halbseitig in das Grundgestrickteil eingestrickt sein.

Das Beinbekleidungsstück nach den vorherigen Ausführungsbeispielen ist bevorzugt als Füßling, Socke, Strumpf, insbesondere Wadenstrumpf, als Strumpfhose, Fußbandage oder als Strickteil einer Fußorthese ausgebildet. Besonders bevorzugt ist es als ein kompressives Beinbekleidungsstück ausgebildet, wobei die Kompressionsstärke in Gestricklängsrichtung, insbesondere von der Fessel in Richtung der Wadenmuskulatur, ab oder zunimmt. Dabei betragen die durch das Beinbekleidungsstück erzeugten kompressiven Drücke in einem Fesselbereich bevorzugt zwischen 10 und 60 mmHg, im Wadenbereich bevorzugt zwischen 5 und 30 mmHg und in einem Mittelfußbereich bevorzugt zwischen 10 und 30 mmHg.

Das vorliegende Beinbekleidungsstück zeichnet sich durch eine Reihe erheblicher Vorteile aus.

Durch die Ausbildung des Beinbekleidungsstück mit einem Fußteil und zumindest einer ersten eingestrickten Funktionszone, die wiederum zur Aufnahme einer Ferse eines Fußes ausgebildet ist und in die zumindest abschnittsweise zumindest ein erster Haftfaden eingestrickt ist, wird ein Beinbekleidungsstück zur Verfügung gestellt, welches in einem Fersenbereich eine rutschhemmende Wirkung aufweist. Dies bringt den Vorteil mit sich, dass beim Gehen bzw. der natürlichen Abrollbewegung des Fußes bereits ein erster Kontakt mit einer externen Oberfläche, also das Aufsetzen der Ferse auf den Fußboden, mit dem rutschfesten Sohlenabschnitt erfolgt. Dadurch wird das Risiko eines Ausrutsches deutlich reduziert.

Auch einen wesentlichen Vorteil der Erfindung bringt die Anordnung und das Hervortreten des zumindest einen eingestrickten ersten Haftfadens an der Gestrickinnen- und Außenseite der ersten Funktionszone mit sich. Das Beinbekleidungsstück gewährleistet dadurch im Bereich der Ferse einen sicheren Halt gegenüber dem Träger als auch einer externen Oberfläche, wie zum Beispiel einem Fußboden oder einem Schuh. Die Gefahr eines Verrutschens des Beinbekleidungsstücks am Fuß eines Trägers, dies auf Grund der Haftwirkung des Beinbekleidungsstücks an bzw. gegenüber der externen Oberfläche, wird dadurch vermieden.

Einen weiteren Vorteil bringt das Einstricken des zumindest einen ersten Haftfadens im Bereich der Fersenunterseite und der Fersenrückseite der ersten Funktionszone mit sich. Neben dem besseren Halt gegenüber dem Fußboden, insbesondere während einer Abrollbewegung des Fußes, wird durch das Einstricken des Haftfadens an der Fersenrückseite das Herausschlüpfen aus einem Schuh vermieden und somit der Halt in einem Schuh deutlich verbessert.

Einen weiteren Vorteil stellt die schritt- bzw. stufenweise Erhöhung oder Reduzierung der Haftwirkung in der ersten Funktionszone des Beinbekleidungsstücks in Umfangsrichtung oder Längsrichtung dar. Ein abrupter Übergang zwischen Abschnitten mit und ohne Haftwirkung wird dadurch vermieden. Dies bringt den Vorteil mit sich, dass die Gefahr des Verrutschens des Grundgestrickteil des Beinbekleidungsstücks an dem Fuß eines Trägers, insbesondere während einer Abrollbewegung des Fußes, deutlich verringert wird.

Schließlich bildet das Einstricken eines zweiten Haftfadens außerhalb der ersten Funktionszone in das Grundgestrickteil, insbesondere an einer zum ersten Haftfaden gegenüberliegenden Seite, um dort eine rutschhemmende Wirkung zu erzeugen, einen weiteren wesentlichen Vorteil. Bevorzugt ist dieser im Bereich des Bundes des Beinbekleidungsstücks eingestrickt. Dies hat den Vorteil, dass zusätzlich der Halt des Beinbekleidungsstücks am Fuß oder Bein eines Trägers verbessert wird.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des Beinbekleidungsstücks, insbesondere in Form eines Wadenstrumpfes, bestehend aus einem Grundgestrickteil mit einer eingestrickten ersten Funktionszone, welche gemäß der Erfindung eine Haftwirkung aufweist, in der Seitenansicht,
Figur 2 ein zweites Ausführungsbeispiel des Beinbekleidungsstücks, insbesondere in Form einer Fußbandage, welche ebenfalls aus einem Grundgestrickteil besteht, welches wiederum ein zweites Ausführungsbeispiel der ersten Funktionszone aufweist, dies erneut in der Seitenansicht,
Figur 3 ein drittes Ausführungsbeispiel des Beinbekleidungsstücks, insbesondere in Form eines Strickteils für eine Fußorthese, mit einem dritten Ausführungsbeispiel der ersten Funktionszone, welche in das Grundgestrickteil eingestrickt ist, dies auch hier in der Seitenansicht,
Figur 4 einen Ausschnitt aus einem weiteren Ausführungsbeispiel des Beinbekleidungsstücks, insbesondere ein viertes Ausführungsbeispiel der eingestrickten ersten Funktionszone, welche zur Aufnahme einer Ferse eines Fußes ausgebildet ist,
Figur 5 schließlich ebenfalls einen Ausschnitt aus einem weiteren Ausführungsbeispiel des Beinbekleidungsstücks, insbesondere ein fünftes Ausführungsbeispiel der eingestrickten ersten Funktionszone,
Figur 6 ein Maschenbild des Beinbekleidungsstücks, insbesondere einen Ausschnitt aus dem zweiten Ausführungsbeispiel der ersten Funktionszone gemäß der Figur 2,
Figur 7A bis 7C drei Maschenbilder des Beinbekleidungsstücks, insbesondere einen Ausschnitt aus dem dritten Ausführungsbeispiel der ersten Funktionszone gemäß der Figur 3,
Figur 8 ein Maschenbild des Beinbekleidungsstücks, insbesondere einen Ausschnitt aus dem vierten Ausführungsbeispiel der ersten Funktionszone gemäß der Figur 4,
Figur 9 schließlich ein Maschenbild des Beinbekleidungsstücks, insbesondere einen Ausschnitt aus dem fünften Ausführungsbeispiel der ersten Funktionszone gemäß der Figur 5;

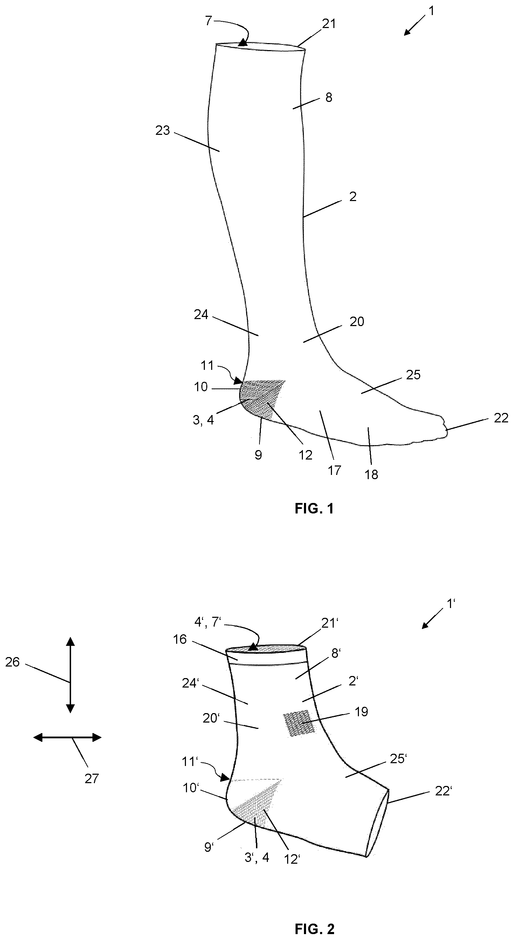

In Figur 1 ist ein erstes Ausführungsbeispiel des Beinbekleidungsstücks, insbesondere in Form eines Wadenstrumpfes 1, in der Seitenansicht gezeigt. Der kompressive Wadenstrumpf 1 besteht dabei aus dem Grundgestrickteil 2, welches neben einem Fußteil 25 auch ein Fesselteil 24 und ein Wadenteil 23 umfasst. Das obere Ende 21 des Grundgestrickteils 2 ist dabei offen ausgebildet. Hier ist die Gestrickinnenseite 7 zu sehen. Das untere Ende 22 ist hingegen geschlossen und bildet dabei in einem Vorfußbereich 18 eine geschlossene Zehenbox aus. In das Grundgestrickteil 2, insbesondere in dessen Fußteil 25, ist nun eine erste Funktionszone 3 eingestrickt, welche zur Aufnahme einer Ferse eines Fußes ausgebildet ist.

Die durch den Wadenstrumpf erzeugten kompressiven Drücke betragen in einem Fesselbereich 20 des Beinbekleidungsstücks 2 zwischen 10 und 60 mmHg, bevorzugt zwischen 10 und 40 mmHg, und in einem Mittelfußbereich 17 zwischen 10 und 30 mmHg. Die Werte werden mittels der eingangs vorgestellten Messmethodik und Messgerät, insbesondere mittels der Prüfung am HOSY Messgerät (Institut Hohenstein), gemessen. Der Wadenstrumpf 1 weist in dem gezeigten Ausführungsbeispiel bevorzugt einen graduellen Kompressionsverlauf auf. D.h. die Kompressionsstärke nimmt hierbei vom Fesselbereich 20 in Richtung oberes Ende 21 ab. Der graduelle Druckverlauf wird durch die Art und Weise der Einbringung eines elastischen Kompressionsfadens, insbesondere eines Schussfadens bestimmt, insbesondere über die Anzahl von aufeinanderfolgenden durch zumindest einen Grundstrickfaden gebildete Maschenreihen, in denen der Schussfaden eingelegt ist.

In die erste Funktionszone 3 zur Aufnahme einer Ferse eines Fußes ist nun zumindest ein erster Haftfaden 4 eingestrickt, um dem Beinbekleidungsstück 1 in einem Fersenbereich eine rutschhemmende Wirkung zu verleihen. Die erste Funktionszone 3 ist bevorzugt als Pendelferse, gestrickt auf einer Rundstrickmaschine, ausgebildet. Der Haftfaden 4 ist dabei bevorzugt auf einen Grundstrickfaden plattiert und zwar derart, dass dieser an der Außenseite 8 des Beinbekleidungsstücks 1 und der Funktionszone 3 hervortritt. Der Haftfaden 4 bildet somit bevorzugt die Außenseite der ersten Funktionszone 3, insbesondere im Bereich der Fersenunterseite 9 sowie der Fersenrückseite 10. Besonders bevorzugt bildet er zudem auch im Bereich der Ferseninnenseite 11 sowie der Fersenaußenseite 12 die Außenseite der ersten Funktionszone 3. Als Haftfaden 4 wird bevorzugt ein Silikon-, Polyurethan-, Polyester-, Elastan- oder Kautschukfaden verwendet.

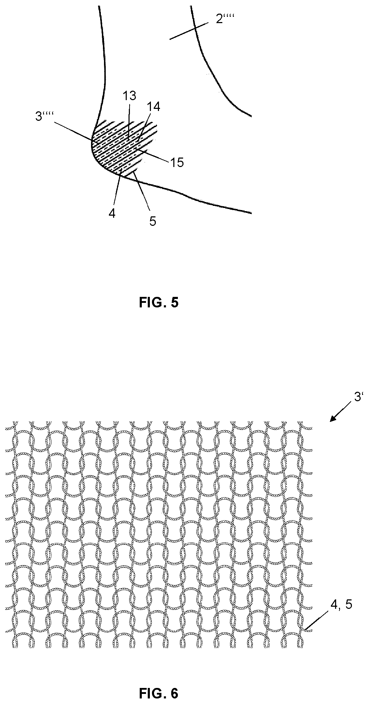

Figur 2 zeigt ein zweites Ausführungsbeispiel des Beinbekleidungsstücks, insbesondere in Form einer Fußbandage 1', welche ebenfalls aus einem Grundgestrickteil 2` besteht. Das Grundgestrickteil 2` umfasst ein Fußteil 25' und ein Fesselteil 24'. Beide Enden 21', 22' des Grundgestrickteils 2' sind dabei offen ausgebildet, wobei das obere Ende 21' einen Bundabschnitt 16 aufweist. Der Bundabschnitt 16 ist dabei bevorzugt zweilagig ausgebildet. Hierzu ist das Grundgestrickteil 2' bevorzugt nach innen gestülpt und dort befestigt. In das Grundgestrickteil 2', insbesondere in dessen Fußteil 25', ist erneut eine erste Funktionszone 3' eingestrickt, welche zur Aufnahme einer Ferse eines Fußes ausgebildet ist. Die Funktionszone 3' erstreckt sich von einer Fersenunterseite 9' bis in eine Fersenrückseite 10`, aber auch in eine Ferseninnenseite 11' und eine Fersenaußenseite 12'. Im Bereich der Fersenunterseite 9' sowie der Ferseninnenseite 11' und der Fersenaußenseite 12' ist nun erfindungsgemäß in die erste Funktionszone 3' ein Haftfaden 4 eingestrickt, um dem Beinbekleidungsstück 1' in einem Fersenbereich eine rutschhemmende Wirkung zu verleihen. Dies insbesondere gegenüber dem Fuß eines Trägers und gleichzeitig gegenüber einer externen Oberfläche. Die erste Funktionszone 3' ist dabei erneut als Pendelferse gestrickt, dies auf einer Rundstrickmaschine. Als Grundstrickfaden wird in der ersten Funktionszone 3' im Bereich der Fersenunterseite 9` nur der Haftfaden 4 verwendet, so dass dieser die Außenseite 8' des Beinbekleidungsstücks 1' und die Innenseite 7' in diesem Bereich bildet. Der übrige Abschnitt der ersten Funktionszone 3', also die zweite obere Hälfte, ist erneut mit dem Grundstrickfaden gestrickt. Die konkrete Maschenkonstruktion wird später in der Figur 6 gezeigt.

Zudem ist ein zweiter Haftfaden 4' im Bereich des Bundabschnitts 16 auf einen Grundstrickfaden plattiert, so dass der zweite Haftfaden 4' in diesem Bereich 16 dem Grundgestrick 2' eine rutschhemmende Wirkung verleiht, um einen besseren Halt der Fußbandage 1' am Fuß oder Bein eines Trägers zu gewährleisten. Als zweiter Haftfaden 4' wird auch in diesem Ausführungsbeispiel bevorzugt ein Silikon-, Polyurethan-, Polyester-, Elastan- oder Kautschukfaden verwendet.

Neben der ersten Funktionszone 3` weist nun das Grundgestrickteil 2' der gezeigten Fußbandage 1' noch eine zweite in das Grundgestrickteil 2' eingestrickte Funktionszone 19 auf. Diese ist bevorzugt im Bereich der Fessel 20, besonders bevorzugt im Bereich eines Knöchels, in das Grundgestrickteil 2` eingestrickt. Diese zweite Funktionszone 19 weist bevorzugt eine höhere Elastizität in Gestricklängs- und/ oder Umfangsrichtung 26, 27 als das Grundgestrickteil 2' auf. Die zweite Funktionszone 19 dient dabei bevorzugt als Entlastungszone.

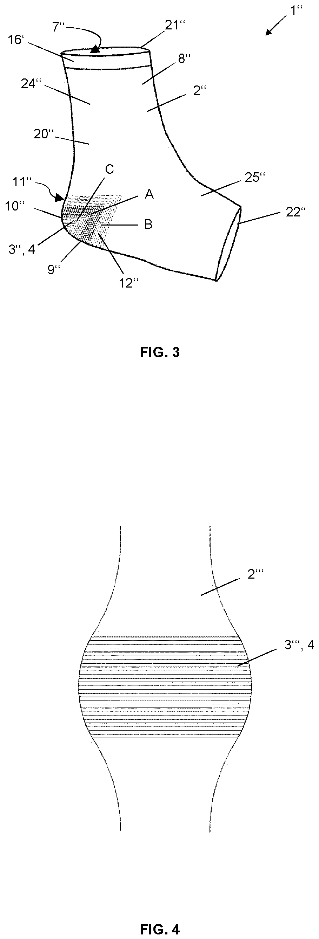

In der Figur 3 ist nun ein drittes Ausführungsbeispiel des Beinbekleidungsstücks, insbesondere in Form eines Strickteils für eine Fußorthese 1", mit einem dritten Ausführungsbeispiel der ersten Funktionszone 3‴, welche in das Grundgestrickteil 2‴ eingestrickt ist, in der Seitenansicht gezeigt. Wie in der Figur 2 bereits gezeigt, weist auch dieses Grundgestrickteil 2" ein Fußteil 25" und ein Fesselteil 24" mit einem bevorzugt kompressiven Fesselbereich 20" auf. Auch hier sind beide Enden 21", 22" des Grundgestrickteil 2" offen ausgebildet. Der Bundabschnitt 16' ist in diesem Ausführungsbeispiel bevorzugt ebenfalls zweilagig ausgebildet.

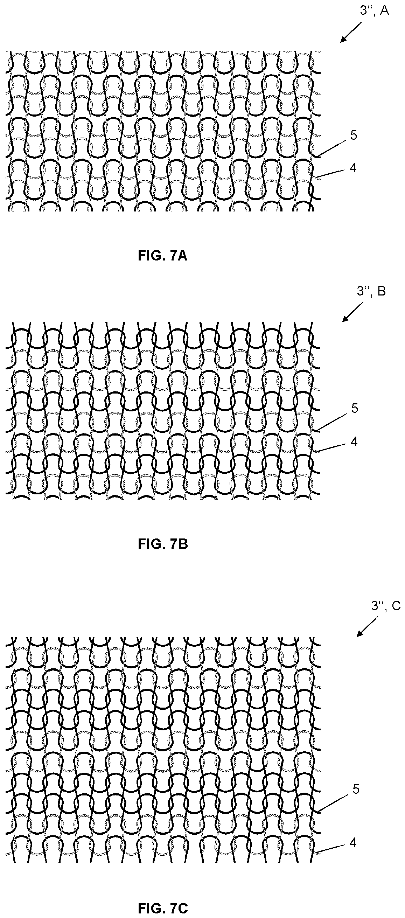

Die erste Funktionszone 3", ebenfalls gebildet als Pendelferse auf einer Rundstrickmaschine, welche zur Aufnahme einer Ferse eines Fußes ausgebildet ist, erstreckt sich auch in diesem Ausführungsbeispiel von einer Fersenunterseite 9" bis in eine Fersenrückseite 10" sowie von einer Ferseninnenseite 11' bis in eine Fersenaußenseite 12". In den zuvor genannten Bereichen 9", 10", 11", 12" der ersten Funktionszone 3" ist nun erfindungsgemäß der erste Haftfaden 4 eingestickt, um dem Beinbekleidungsstück 1" in einem Fersenbereich eine rutschhemmende Wirkung zu verleihen. Der Haftfaden 4 ist dabei bevorzugt im Bereich der ersten Funktionszone 3" als zweiter grundstrickmaschenbildender Faden eingestrickt. Dadurch bildet dieser abschnittsweise die Außenseite 8" und Innenseite 7" des Beinbekleidungsstücks 1". Die Maschenkonstruktion wird später in den Figuren 7A bis 7C im Detail beschrieben.

Wie bereits auch zu Figur 2 beschrieben, ist bevorzugt ebenfalls ein zweiter Haftfaden 4" im Bereich des Bundabschnitts 16" auf einen Grundstrickfaden plattiert, so dass der zweite Haftfaden 4" in diesem Bereich 16 an der Innenseite 7" des Grundgestrickteils 2" dem Grundgestrick 2" eine rutschhemmende Wirkung verleiht. Der erste Haftfaden 4 und der zweite Haftfaden 4" treten somit an den gegenüberliegenden Seiten des Grundgestrickteils 2" hervor.

Des Weiteren ist der Figur 3 zu entnehmen, dass gemäß diesem Ausführungsbeispiel die erste Funktionszone 3" die drei Zonen A, B und C umfasst. Gemäß der Erfindung kann nämlich die Menge des zumindest einen ersten Haftfadens 4 innerhalb der ersten Funktionszone 3" in Gestricklängs- und/ oder Umfangsrichtung, die insbesondere aus dem Grundgestrickteil 2" hervortritt, variieren. Dadurch ist es möglich die Haftwirkung schritt- bzw. stufenweise zu erhöhen oder zu reduzieren. So ist bevorzugt in Zone A die meiste Menge an Haftfaden eingestrickt, gefolgt von der Zone B. In Zone C ist hingegen die geringste Menge an Haftfaden verstrickt. Zumindest tritt in Zone C die geringste Menge an der Oberfläche hervor. Ein abrupter Übergang zwischen Abschnitten mit und ohne Haftwirkung wird dadurch vermieden.

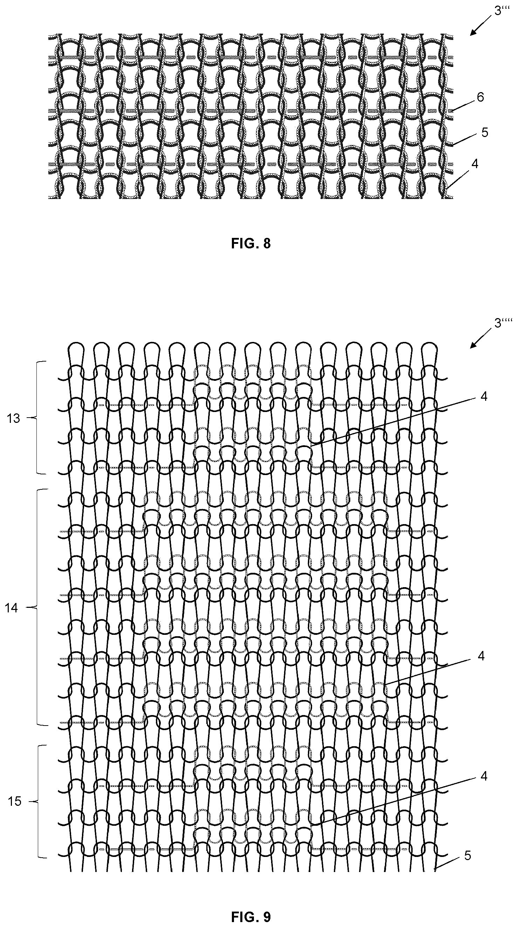

Die Figur 4 zeigt nun einen Ausschnitt aus einem weiteren Ausführungsbeispiel des Beinbekleidungsstücks, insbesondere ein viertes Ausführungsbeispiel der eingestrickten ersten Funktionszone 3‴, welche zur Aufnahme einer Ferse eines Fußes ausgebildet ist. Gemäß diesem Ausführungsbeispiel ist die erste Funktionszone 3‴ als sogenannte Schlauchferse ausgebildet, die in das Grundgestrickteil 2‴ eingestrickt ist. Die Fersenform wird hierbei durch die Vergrößerung der Grundstrickmaschen und dem kompressionsgebenden Faden, insbesondere einem Schussfaden, der mit weniger Vorspannung in das Grundgestrickteil eingestrickt, gebildet. Die erste Funktionszone 3‴ ist dabei umlaufend ausgebildet und vergrößert dadurch den Durchmesser des Grundgestrickteils 2‴. In die Schlauchferse ist nun erfindungsgemäß ein Haftfaden 4 eingearbeitet. Die konkrete Maschenkonstruktion wird später in der Figur 8 im Detail beschrieben.

In Figur 5 ist schließlich ebenfalls ein Ausschnitt aus einem weiteren Ausführungsbeispiel des Beinbekleidungsstücks, insbesondere ein fünftes Ausführungsbeispiel der eingestrickten ersten Funktionszone 3"" gezeigt. Gemäß diesem Ausführungsbeispiel ist die erste Funktionszone 3"" durch in das Grundgestrickteil 2‴, insbesondere zwischen zwei Grundstrickmaschenreihen, welche mit einem Grundstrickfaden 5 gestrickt sind, eingestrickte Teilmaschenreihen gebildet. Dabei werden die zusätzliche Teilmaschenreihen bevorzugt durch den ersten Haftfaden 4 gebildet, so dass diese Teilmaschenreihen Antirutschelemente 13, 14, 15 bilden. Die Maschenkonstruktion wird später in der Figur 9 im Detail beschrieben.

Das Maschenbild gemäß Figur 6 zeigt einen Ausschnitt aus dem zweiten Ausführungsbeispiel der ersten Funktionszone 3` gemäß der Figur 2. Das Maschenbild zeigt, dass in der ersten Funktionsszone 3', welche als Pendelferse ausgebildet ist, ein Grundstrickfaden 5 bzw. der Haftfaden 4, welcher den Grundstrickfaden 5 in diesem Gestrickabschnitt ersetzt, maschenbildend über mehrere Strickreihen und Maschenstäbchen hinweg verstrickt ist. Es ist kein weiterer Faden, insbesondere auch kein kompressionsgebender Faden eingestrickt. Durch diese Ausbildung der ersten Funktionsszone 3' bildet der Haftfaden 4 in diesem Bereich sowohl die Innenseite als auch die Außenseite der Funktionszone 3', was dem Gestrick in diesem Fersenbereich eine rutschhemmende Wirkung verleiht, insbesondere gegenüber dem Fuß eines Trägers, aber auch gleichzeitig gegenüber einer externen Oberfläche.

Die Figuren 7A bis 7C zeigen nun drei Maschenbilder des Beinbekleidungsstücks, insbesondere einen Ausschnitt aus dem dritten Ausführungsbeispiel der ersten Funktionszone 3" gemäß der Figur 3. Die erste Funktionszone 3" ist bevorzugt als Pendelferse auf einer Rundstrickmaschine gebildet. Neben dem Grundstrickfaden 5, welcher über eine oder mehrere Maschenreihen hinweg verstrickt ist, weist die Funktionszone 3" einen Haftfaden 4 auf, welcher ebenfalls Grundstrickmaschen bildet und über bevorzugt eine ganze Maschenreihe hinweg verstrickt ist. Der Haftfaden 4 ist somit im Bereich der ersten Funktionszone 3" als zweiter Grundstrickfaden maschenbildend zwischen zwei Grundstrickmaschenreihen eingestrickt. Dadurch bildet dieser abschnittsweise die Außenseite 8" des Beinbekleidungsstücks 1".

Gemäß der Erfindung variiert nun die Menge des zumindest einen ersten Haftfadens 4 innerhalb der ersten Funktionszone 3" in Gestricklängsrichtung, dies insbesondere in den drei Zonen A, B und C. Dabei zeigt die Figur 7A einen Ausschnitt aus der Zone A, die Figur 7B einen Ausschnitt aus der Zone B und schließlich die Figur 7C einen Ausschnitt aus der Zone C.

Wie die Figur 7A zeigt, wechseln sich in der ersten Funktionszone 3" in der Zone A der Grundstrickfaden 5 und der Haftfaden 4 von Maschenreihe zu Maschenreihe ab. Jede zweite Grundstrickmaschenreihe bilde somit eine Haftreihe, um dem Gestrick eine haftende Eigenschaft zu verleihen. Das Maschenbild in der Figur 7B zeigt, dass in der ersten Funktionszone 3" in der Zone B erst nach zwei Grundstrickmaschenreihen, gebildet durch den Grundstrickfaden 5, eine Maschenreihe gebildet durch den Haftfaden 4 folgt. D.h. die Zone B weist eine geringere Haftwirkung, als die Zone A auf. Schließlich zeigt die Figur 7C ein Maschenbild wonach sich in der ersten Funktionszone 3" in der Zone C nach drei durch den Grundstrickfaden 5 gebildete Grundstrickmaschenreihen eine Maschenreihe gebildet durch den Haftfaden 4 folgt.

Somit weist die Zone C im Vergleich zu den Zonen A und B die geringste Haftwirkung auf. Dadurch ist es möglich die Haftwirkung schritt- bzw. stufenweise zu erhöhen oder reduzieren. Ein abrupter Übergang zwischen Abschnitten mit und ohne Haftwirkung wird dadurch jedenfalls vermieden.

Das Maschenbild gemäß Figur 8 zeigt nun einen Ausschnitt aus dem vierten Ausführungsbeispiel der ersten Funktionszone 3‴ gemäß der Figur 4, wonach die erste Funktionszone 3‴ als Schlauchferse ausgebildet ist. Das Maschenbild zeigt, dass in der ersten Funktionszone 3‴ der Grundstrickfaden 5 maschenbildend über mehrere Strickreihen und Maschenstäbchen hinweg verstrickt ist. Auf den Grundstrickfaden 5 ist vollflächig, also auf jede durch den Grundstrickfaden 5 gebildete Masche, der Haftfaden 4 plattiert. Dies insbesondere derart, dass dieser an der Außenseite des Gestricks zum Liegen kommt. In jeder zweiten Maschenreihe ist ferner ein Kompressionsfaden, insbesondere ein Schussfaden 6, über Fang und Flottung in die durch den grundstrickfaden 5 gebildete Grundstrickmaschen eingelegt, um der ersten Funktionszone 3" neben einer rutschhemmenden Wirkung auch eine kompressive Wirkung zu verleihen.

In der Figur 9 ist nun ein Maschenbild eines Ausschnitts aus dem fünften Ausführungsbeispiel der ersten Funktionszone 3"" gemäß der Figur 5 gezeigt. Das Maschenbild zeigt, dass in der ersten Funktionszone 3"" der Grundstrickfaden 5 maschenbildend über mehrere Strickreihen und Maschenstäbchen hinweg verstrickt ist. Zwischen die Grundstrickmaschen und Grundstrickmaschenreihen ist nun erfindungsgemäß ein Zusatzfaden, insbesondere der Haftfaden 4, unter Bildung von Zusatzmaschen und Teilmaschenreihen mit unterschiedlicher Länge eingestrickt. In dem Abschnitt 13 bildet der Haftfaden zwischen zwei Grundstrickmaschenreihen eine Teilmaschenreihe mit einer Länge von fünf Zusatzmaschen. In Abschnitt 13 beträgt die Länge der Teilmaschenreihen zehn Zusatzmaschen. In Abschnitt 15 werden durch den Haftfaden 4 erneut fünf Zusatzmaschen gebildet. Der Haftfaden wird dabei am Anfang und am Ende durch die Verriegelungstechnik Fang und Flottung in den Grundstickmaschenreihen befestigt. In Gestricklängsrichtung gesehen, umfassen die Abschnitte 13 und 15 jeweils zwei Zusatzmaschenreihen. Abschnitt 14 hingegen ist durch vier Zusatzmaschenreihen gebildet. Die Abschnitte 13, 14 und 15 bilden somit mehrere Antirutschelemente aus, welche dem Gestrick in diesem Fersenbereich eine rutschhemmende Wirkung verleihen, insbesondere gegenüber dem Fuß eines Trägers, aber auch gleichzeitig gegenüber einer externen Oberfläche.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Beinbekleidungsstück (1, 1', 1") aufweisend ein zumindest ein Fußteil ausbildendes Grundgestrickteil (2, 2', 2", 2‴, 2"") mit zumindest einer ersten in das Grundgestrickteil (2, 2', 2", 2‴, 2"") eingestrickten Funktionszone (3, 3', 3", 3‴, 3""), welche zur Aufnahme einer Ferse eines Fußes ausgebildet ist, **dadurch gekennzeichnet, dass** in die erste Funktionszone (3, 3', 3", 3‴, 3"") zumindest abschnittsweise zumindest ein erster Haftfaden (4) eingestrickt ist, um dem Beinbekleidungsstück (1, 1', 1") in einem Fersenbereich eine rutschhemmende Wirkung zu verleihen.

2. Beinbekleidungsstück (1, 1', 1") nach Anspruch 1, **dadurch gekennzeichnet, dass** der Haftfaden (4) ein Silikon-, Polyurethan-, Polyester-, Elastan- oder Kautschukfaden ist.

3. Beinbekleidungsstück (1, 1', 1") nach Anspruch 1 oder 2, **dadurch gekennzeichnet, dass** der zumindest eine eingestrickte erste Haftfaden (4) an der Gestrickinnen- und/ oder Außenseite (7, 7', 7"; 8, 8', 8") der ersten Funktionszone (3, 3', 3", 3‴, 3"") hervortritt.

4. Beinbekleidungsstück (1, 1', 1") nach einem der vorherigen Ansprüche, **dadurch gekennzeichnet, dass** der zumindest eine erster Haftfaden (4) zumindest abschnittsweise im Bereich der Fersenunterseite (9, 9', 9") und/ oder der Fersenrückseite (10', 10") in die erste Funktionszone (3, 3', 3", 3‴, 3"") eingestrickt ist.

5. Beinbekleidungsstück (1, 1', 1") nach einem der vorherigen Ansprüche, **dadurch gekennzeichnet, dass** der zumindest eine erster Haftfaden (4) im Bereich der Ferseninnenseite (11, 11', 11") und/ oder der Fersenaußenseite (12, 12', 12") in die erste Funktionszone (3, 3', 3", 3‴, 3"") eingestrickt ist.

6. Beinbekleidungsstück (1") nach einem der vorherigen Ansprüche, **dadurch gekennzeichnet, dass** die Menge des zumindest einen ersten Haftfaden (4) in der ersten Funktionszone (3", 3"") in Gestricklängs- und/ oder Umfangsrichtung (26, 27) variiert.

7. Beinbekleidungsstück nach einem der vorherigen Ansprüche, **dadurch gekennzeichnet, dass** der Haftfaden (4) zur Bildung eines oder mehrerer Antirutschelemente (13, 14, 15) lokal begrenzt an der Gestrickinnen- und/ oder Außenseite (8, 8', 8") der ersten Funktionszone (3"") hervortritt.

8. Beinbekleidungsstück (1') nach einem der vorherigen Ansprüche, **dadurch gekennzeichnet, dass** in das Grundgestrickteil (2`) außerhalb der ersten Funktionszone (3`) zumindest ein zweiter Haftfaden (4', 4") eingestrickt ist, um dem Beinbekleidungsstück (1'), bevorzugt in einem Bundabschnitt (16), eine rutschhemmende Wirkung zu verleihen.

9. Beinbekleidungsstück (1') nach einem der vorherigen Ansprüche, **dadurch gekennzeichnet, dass** der zumindest eine erste Haftfaden (4) und der zumindest eine zweite Haftfaden (4`) an den gegenüberliegenden Seiten (7, 7', 7"; 8, 8', 8") des Grundgestrickteils (2`) hervortreten.

10. Beinbekleidungsstück (1') nach einem der vorherigen Ansprüche, **dadurch gekennzeichnet, dass** das Grundgestrickteil (2`) zumindest eine zweite in das Grundgestrickteil (2`) eingestrickte Funktionszone (19), welche bevorzugt eine höhere Elastizität in Gestricklängs- und/ oder Umfangsrichtung (26, 27) als das Grundgestrickteil (2`) aufweist, umfasst.

11. Beinbekleidungsstück (1) nach einem der vorherigen Ansprüche, **dadurch gekennzeichnet, dass** das Grundgestrickteil (2) durch zumindest einen maschenbildenden Grundstrickfaden (5) sowie zumindest einen eingelegten und/ oder verstrickten elastischen Kompressionsfaden (6) gebildet ist.

12. Beinbekleidungsstück (1) nach Anspruch 11, **dadurch gekennzeichnet, dass** die durch das Grundgestrickteil (2) erzeugten kompressiven Drücke in einem Fesselbereich (20) des Beinbekleidungsstücks (1) zwischen 10 und 60 mmHg betragen.

13. Beinbekleidungsstück (1, 1', 1") nach einem der vorherigen Ansprüche, **dadurch gekennzeichnet, dass** die zumindest eine erste Funktionszone (3, 3', 3", 3‴, 3"") durch zumindest einen maschenbildenden Grundstrickfaden (5) und den zumindest einen ersten Haftfaden (4) oder durch den zumindest einen ersten Haftfaden (4) alleine gebildet ist.

14. Beinbekleidungsstück (1, 1', 1") nach Anspruch 13, **dadurch gekennzeichnet, dass** die durch den zumindest einen Grundstrickfaden (5) gebildete erste Funktionszone (3, 3', 3", 3‴) als Pendelferse, Schlauchferse oder durch einen oder mehrere Spickel ausgebildet ist, wobei der zumindest eine erste Haftfaden (4) auf den zumindest einen Grundstrickfaden (5) zumindest abschnittsweise plattiert ist oder dass in die durch den zumindest einen Grundstrickfaden (5) gebildete erste Funktionszone (3"") mehrere durch den zumindest einen Haftfaden (4) gebildete Teilmaschenreihen (13, 14, 15) eingestrickt sind.

15. Beinbekleidungsstück (1, 1', 1") nach einem der vorherigen Ansprüche, **dadurch gekennzeichnet, dass** das Beinbekleidungsstück (1, 1', 1") ein Füßling, eine Socke, ein Strumpf, insbesondere ein Wadenstrumpf (1), eine Strumpfhose, eine Fußbandage (1') oder ein Strickteil einer Fußorthese (1") ist.
